# EUROPEAN PATENT SPECIFICATION

(11) **EP 3 721 933 B1**
(45) Date of publication and mention of the grant of the patent: **21.02.2024**
(21) Application number: 20167438.9
(22) Date of filing: 31.03.2020
(51) Int. Cl.: A61M 25/06, A61M 25/00, A61B 1/005, A61B 1/00

(54) **BENDABLE URETERAL ACCESS SHEATH**
BIEGSAME URETERZUGANGSHÜLLE
GAINE D'ACCÈS URÉTÉRAL PLIABLE

(30) Priority: 03.04.2019 CN 201920466208 U
(43) Date of publication of application: 14.10.2020
(73) Proprietor: Guangxi Medical Association, Nanning 530021 (CN)
(72) Inventor: Cheng, Jiwen, Nanning, 530021 (CN); Li, Tianyu, Nanning, 530021 (CN); Mo, Zengnan, Nanning, 530021 (CN)
(74) Representative: Wang, Bo

(56) References cited:
- WO-A1-2010/132560
- WO-A2-2007/081706
- US-B1- 9 782 566

## Description

### FIELD

The present disclosure relates to the technical field of medical devices for the urinary system. In particular, provided herein is a bendable ureteral access sheath for a flexible ureteroscope.

### BACKGROUND

Clinically, a ureteral access sheath is placed into a ureter before retrograde flexible ureteroscopy to provide an access to the ureter for a flexible ureteroscope and to provide real-time drainage of lavage fluid to maintain a clear view field and to reduce intrapelvic pressure. A traditional ureteral access sheath is straight and kink-resistant, which may lead to the damage of the ureteral access sheath when passing through a curvature (especially at a ureteropelvic junction) during surgery, and which may lead to ureteral perforation and urinary extravasation. A patient who has undergone multiple surgeries on the ureter or adjacent organs, or a patient with a large curvature at the ureteropelvic junction is prone to ureteral perforation or misplacement of the ureteral access sheath, thus resulting in inadequate drainage of lavage fluid in operation, excessive intrapelvic pressure, urosepsis, etc. Therefore, it is of clinical significance to have a ureteral access sheath with the ability to bend and change direction freely.

See, e.g., WO2010132560 A1, WO2007081706 A2 and US9782566 B1.

### SUMMARY OF THE INVENTION

The invention is defined by independent claim 1 and subsequent dependent claims 2 - 5.

### SUMMARY OF THE DISCLOSURE

Provided herein is a bendable ureteral access sheath for a flexible ureteroscope, which overcomes the problems of a traditional ureteral access sheath as being kink-resistant to cause damages to a ureter and avoids inadequate drainage of lavage fluid in operation due to misplacement. Also, the bendable ureteral access sheath provided herein can improve the efficiency and safety of flexible ureteroscopy.

The inventive device comprises a bendable ureteral access sheath comprising a sheath tube, an inner tube, and a sheath tube hub; wherein the sheath tube is sleeved on the outside of the inner tube; wherein the sheath tube has a tail end that is connected to the sheath tube hub; and the sheath tube comprises a first straight tube section, a bendable tube section, and a second straight tube section, wherein the first straight tube section, bendable tube section, and second straight tube section are connected sequentially to each other; wherein the first and second straight tube sections each independently comprise reinforcing steel wires placed in their tube walls and thus are each kink-resistant; and wherein the bendable tube section is bendable.

In the above embodiment, the bendable tube section of the ureteral sheath tube can be bent freely. After a zebra guidewire is placed into a ureter, the sheath tube can move inwards along the zebra guidewire. When encountering a physiological or pathological curvature, the bendable tube section can change direction and bend adequately to ensure that the sheath is pushed in without directly scratching and puncturing a ureteral mucous layer, a ureteral muscle layer, or a ureteral wall, thereby effectively protecting the ureter.

To achieve a better bending effect, the inventive bendable tube section described herein has a flexible and foldable corrugated tube structure.

The inventive bendable tube section described herein comprises annular reinforcing steel rings in the corrugated tube structure.

In certain embodiments, the first and second straight tube sections each comprise reinforcing steel wires in their tube walls configured longitudinally at equal intervals.

In certain embodiments, the sheath tube is made from a plastic material *via* injection molding and has both its inner and outer walls coated with hydrophilic coatings.

In certain embodiments, the inner tube is made of a polytetrafluoroethylene (*e.g*., TEFLON) with certain bendability.

Preferably, the bendable tube section has a length ranging from 1 to 2 cm and the first straight tube section has a length ranging from 2 to 3 cm.

The bendable ureteral access sheath provided herein has the following advantages and effects.

First, the bendable ureteral access sheath comprising a bendable tube section with an appropriate length, together with the first and second straight tube sections each reinforced with the reinforcing steel wires, while ensuring the thrust force during a pushing-in process along the zebra guidewire together with the inner tube, can easily cross a physiological or pathological curvature of the ureter because of the flexibility of the bendable tube section, thereby leading to a smooth upward movement and preventing direct damages to the ureteral mucous layer and ureteral wall muscle layer caused by a traditional ureteral access sheath having a straight and kink-resistant structure and allowing a simple single direction movement only.

Secondly, the first and second straight tube sections are provided with built-in reinforcing steel wires, and thus imaging and positioning can be performed by intraoperative X-rays during surgery. The built-in reinforcing steel wires also provide good support for a ureteral lumen.

Thirdly, the distal end of the sheath tube having the bendable tube section reaches the ureteropelvic junction more easily to drain turbid lavage liquid, urine, and powered stones in real-time during operation, thereby preventing severe complications such as urosepsis, infectious shock, and even death caused by intrarenal backflow due to high intrapelvic pressure. At the same time, by improving the surgical field of vision, the efficiency of laser lithotripsy under flexible microscopy can be effectively improved, and the mucosal damage and bleeding of the renal pelvis and calyx can be reduced.

### BRIEF DESCRIPTION OF THE DRAWINGS

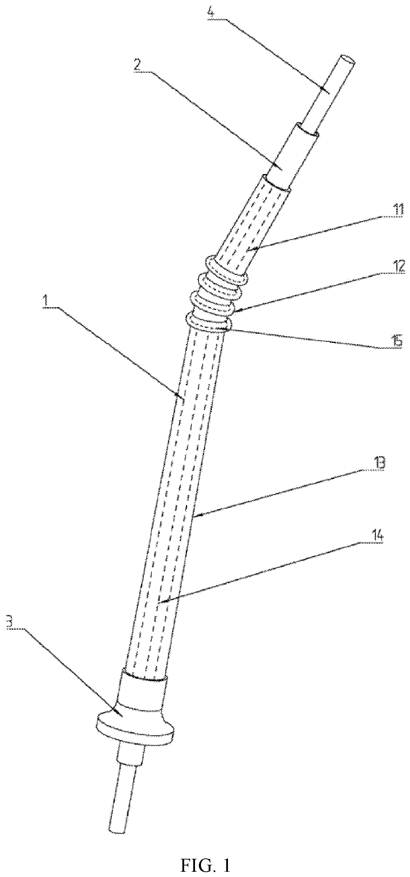

FIG. 1 is a schematic diagram of a sheath tube of a bendable ureteral access sheath for a flexible ureteroscope. In the figure, 1: a sheath tube; 11: a first straight tube section; 12: a bendable tube section; 13: a second straight tube section; 14: reinforcing steel wires; 15: annular reinforcing steel rings; 2: an inner tube; 3: a sheath tube hub; and 4: a zebra guidewire.

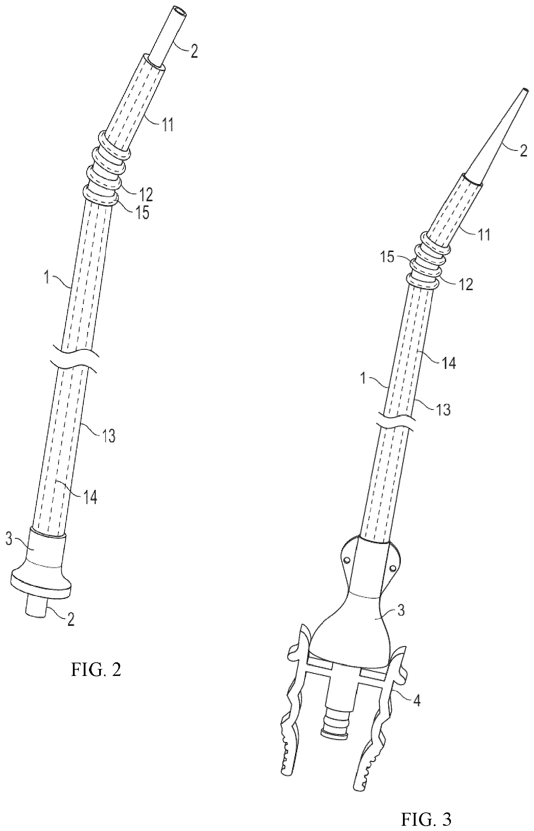

FIG. 2 is a schematic diagram of a sheath tube of a bendable ureteral access sheath, where the sheath tube 1 having a sheath tube hub 3 attached to its tail end comprises a first straight tube section 11, a bendable tube section 12, a second straight tube section 13, reinforcing steel wires 14, and annular reinforcing steel rings 15, where the first and second straight tube sections (11 and 13) are each reinforced with the reinforcing steel wires 14 in their walls, and the bendable tube section 12 is reinforced with the annular reinforcing steel rings 15 in its wall.
FIG. 3 is a schematic diagram of a bendable ureteral access sheath, comprising:
   (i) a sheath tube 1 having a sheath tube hub 3 attached to its tail end, comprising a first straight tube section 11, a bendable tube section 12, a second straight tube section 13, reinforcing steel wires 14, and annular reinforcing steel rings 15, where the first and second straight tube sections (11 and 13) are each reinforced with the reinforcing steel wires 14 in their walls, and the bendable tube section 12 is reinforced with the annular reinforcing steel rings 15 in its wall; and
   (ii) an inner tube 2, which can functions as a dilator or obturator, having a tapered tip on its front end and an inner tube knob 4 attached to its tail end; where the inner tube 2 is fully engaged with the sheath tube 1 *via* the sheath tube hub 3 and the inner tube knob 4.

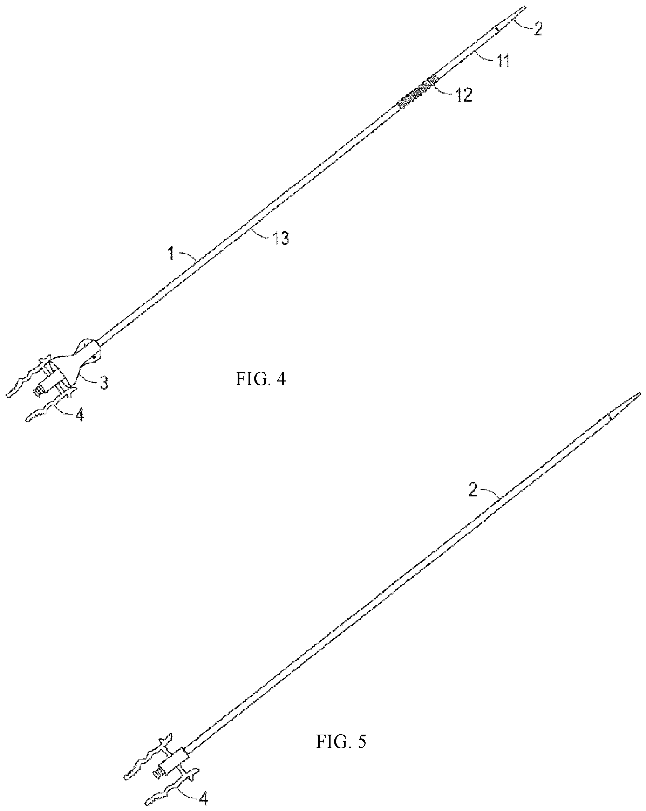

FIG. 4 is a schematic diagram of the bendable ureteral access sheath of FIG. 3, comprising: (i) a sheath tube 1 having a sheath tube hub 3 attached to its tail end, comprising a first straight tube section 11, a bendable tube section 12, and a second straight tube section 13; and (ii) an inner tube 2 having a tapered tip on its front end and an inner tube knob 4 attached to its tail end; where the inner tube 2 is fully engaged with the sheath tube 1 *via* the sheath tube hub 3 and the inner tube knob 4.
FIG. 5 is a schematic diagram of an inner tube 2 of the bendable ureteral access sheath of FIG. 3, having a tapered tip on its front end and an inner tube knob 4 attached to its tail end.

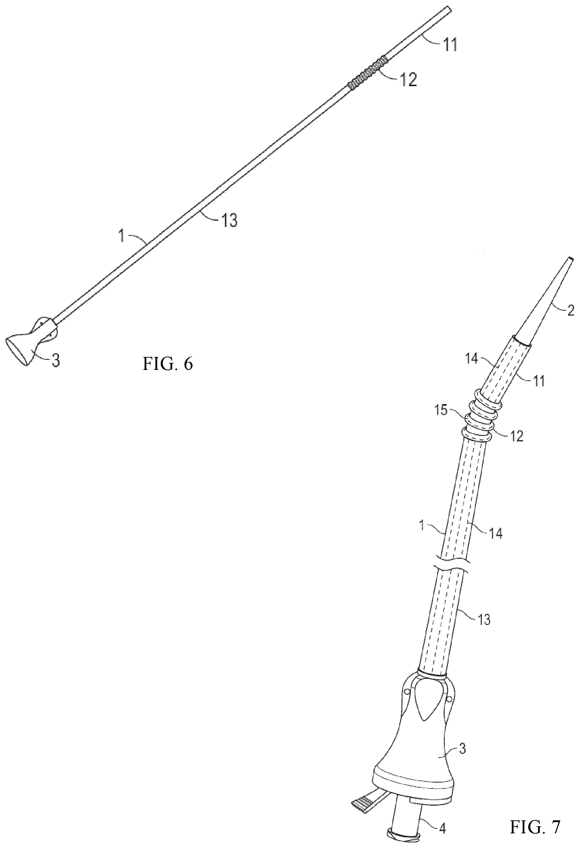

FIG. 6 is a schematic diagram of a sheath tube 1 of the bendable ureteral access sheath of FIG. 3, comprising a first straight tube section 11, a bendable tube section 12, a second straight tube section 13, and a sheath tube hub 3, where the sheath tube hub 3 is attached to the tail end of the sheath tube 1.
FIG. 7 is a schematic diagram of a bendable ureteral access sheath, comprising: (i) a sheath tube 1 having a sheath tube hub 3 attached to its tail end, comprising a first straight tube section 11, a bendable tube section 12, a second straight tube section 13, reinforcing steel wires 14, and annular reinforcing steel rings 15, where the first and second straight tube sections (11 and 13) are each reinforced with the reinforcing steel wires 14 in their walls and the bendable tube section 12 is reinforced with the annular reinforcing steel rings 15 in its wall; and (ii) an inner tube 2 having a tapered tip on its front end and an inner tube knob 4 attached to its tail end; where the inner tube 2 is fully engaged with the sheath tube 1 *via* the sheath tube hub 3 and the inner tube knob 4.

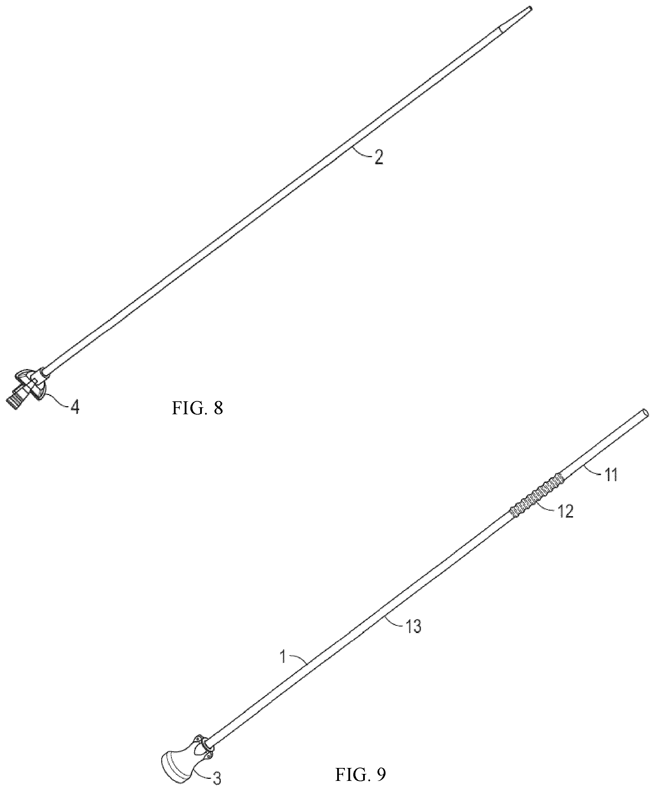

FIG. 8 is a schematic diagram of an inner tube 2 of the bendable ureteral access sheath of FIG. 7, having a tapered tip on its front end and an inner tube knob 4 attached to its tail end.
FIG. 9 is a schematic diagram of a sheath tube 1 of the bendable ureteral access sheath of FIG. 7, comprising a first straight tube section 11, a bendable tube section 12, a second straight tube section 13, and a sheath tube hub 3, where the sheath tube hub 3 is attached to the tail end of the sheath tube 1.

### DETAILED DESCRIPTION

The disclosure will be further understood by the following non-limiting examples.

According to a bendable ureteral access sheath of the example as shown in FIG. 1, the bendable ureteral access sheath comprises a sheath tube 1, an inner tube 2, and a sheath tube hub 3. The sheath tube 1 is sleeved on the outside of the inner tube 2. The sheath tube hub 3 is connected to the tail end of the sheath tube 1. The inner tube 2 has a lumen for a zebra guidewire 4 to pass through. The front end of the inner tube 2 is longer than the sheath tube 1 and is extended out beyond the front end of the sheath tube 1.

The sheath tube 1 comprises a first straight tube section 11, a bendable tube section 12, and a second straight tube section 13 connected sequentially, wherein the second straight tube section 13 is longer than the first straight tube section 11. The tube walls of the first straight tube section 11 and the second straight tube section 13 are reinforced with reinforcing steel wires 14 longitudinally at equal intervals. The reinforcing steel wires 14 can enhance the strength of the sheath tube 1 to provide good support for the ureteral lumen and allow imaging and positioning by intraoperative X-rays during surgery to increase the efficiency of operation.

The bendable tube section 12 has a flexible and foldable corrugated tube structure, wherein annular reinforcing rings 15 are placed in the flexible and foldable corrugated tube structure. The annular reinforcing rings 15 can ensure a liquid passageway of the bendable tube section 12 clear by preventing the bendable tube section 12 from being squeezed and blocked by the ureteral lumen to affect the drainage as shown in FIG. 1.

The length of the first straight tube section 11 is preferably 3 cm and the length of the bendable tube section 12 is preferably 2 cm. The bendable ureteral access sheath having the bendable tube section 12 and the first straight tube section 11 with such lengths is more suitable for placement and drainage at a ureteropelvic junction.

The working principle of the present disclosure is as follows.

A zebra guidewire 4 is placed in the ureter under the direct observation *via* a rigid ureteroscope. A bendable ureteral access sheath is pushed into the ureter along the zebra guidewire 4. During the pushing process, the first straight tube section 11, the bendable tube section 12, and the second straight tube section 13 are all supported and moved forwards by the inner tube 2 of the bendable ureteral access sheath.

When encountering a physiological or pathological curvature, the first straight tube section 11 moves up along the pre-retained zebra guidewire 4, and the bendable tube section 12 can appropriately bend and change direction to continue moving up along with the first straight tube section 11 without directly scratching and puncturing a ureteral mucous layer, a ureteral muscle layer, or a ureteral wall during placement, thereby effectively protecting the ureteral structure.

FIGS. 3, 4, and 7 each show a bendable ureteral access sheath with an inner tube 2 fully engaged with a sheath tube 1, where the tapered tip of the inner tube 2 extended out of the front end of the sheath tube 1 to ease the insertion of the bendable ureteral access into a ureter. Once the bendable ureteral access sheath is in a desired location, the inner tube 2 is removed to provide a working channel for a flexible ureteroscope.

The sheath tube 1 has a front end and a tail end. The distance between the front and tail ends defines the length of the sheath tube 1. In certain embodiments, the sheath tube 1 has a length ranging from about 20 to about 60 cm, from about 20 to about 55 cm, from about 30 to about 50 cm, or from about 30 to about 45 cm. In certain embodiments, the sheath tube 1 has a length ranging from about 20 to about 60 cm. In certain embodiments, the sheath tube 1 has a length ranging from about 20 to about 55 cm. In certain embodiments, the sheath tube 1 has a length ranging from about 30 to about 50 cm. In certain embodiments, the sheath tube 1 has a length ranging from about 30 to about 45 cm. In certain embodiments, the sheath tube 1 has a length of about 35, about 37, about 40, about 42, or about 45 cm.

In certain embodiments, the sheath tube 1 is made from a plastic material. In certain embodiments, the sheath tube 1 is made from a fluorinated ethylene propylene (FEP) copolymer, latex, a nylon, a polyether block amide, a polyethylene, a polyethylene terephthalate (PET), a polytetrafluoroethylene (PTFE), a polyurethane, or a silicone.

In certain embodiments, the first straight tube section 11 has a length ranging from about 15 to about 55 cm, from about 25 to about 55 cm, from about 30 to about 45 cm, from about 30 to about 42 cm, or from about 30 to about 40 cm. In certain embodiments, the first straight tube section 11 has a length ranging from about 15 to about 55 cm. In certain embodiments, the first straight tube section 11 has a length ranging from about 25 to about 55 cm. In certain embodiments, the first straight tube section 11 has a length ranging from about 30 to about 45 cm. In certain embodiments, the first straight tube section 11 has a length ranging from about 30 to about 42 cm. In certain embodiments, the first straight tube section 11 has a length ranging from about 30 to about 40 cm. In certain embodiments, the first straight tube section 11 has a length of about 30, about 32, about 35, about 37, about 40, about 42, or about 45 cm.

In certain embodiments, the first straight tube section 11 has an inner diameter (I.D.) ranging from about 8 to about 20 French, from about 10 to about 15 French, or from about 11 to about 14 French. In certain embodiments, the first straight tube section 11 has an I.D. ranging from about 8 to about 20 French. In certain embodiments, the first straight tube section 11 has an I.D. ranging from about 10 to about 15 French. In certain embodiments, the first straight tube section 11 has an I.D. ranging from about 11 to about 14 French. In certain embodiments, the first straight tube section 11 has an I.D. of about 11, about 12, about 13, or about 14 French.

In certain embodiments, the first straight tube section 11 has an outer diameter (O.D.) ranging from about 9 to about 22 French, from about 11 to about 17 French, or from about 12 to about 16 French. In certain embodiments, the first straight tube section 11 has an O.D. ranging from about 9 to about 22 French. In certain embodiments, the first straight tube section 11 has an O.D. ranging from about 11 to about 17 French. In certain embodiments, the first straight tube section 11 has an O.D. ranging from about 12 to about 16 French. In certain embodiments, the first straight tube section 11 has an O.D. of about 12, about 13, about 14, about 15, or about 16 French.

In certain embodiments, the bendable tube section 12 has a length that is no greater than that of the second straight tube section 13. In certain embodiments, the bendable tube section 12 has a length that is less than that of the second straight tube section 13.

In certain embodiments, the bendable tube section 12 has a length ranging from about 1 to about 5 cm, from about 1 to about 4 cm, from about 1 to about 3 cm, or from about 1 to about 2 cm. In certain embodiments, the bendable tube section 12 has a length ranging from about 1 to about 5 cm. In certain embodiments, the bendable tube section 12 has a length ranging from about 1 to about 4 cm. In certain embodiments, the bendable tube section 12 has a length ranging from about 1 to about 3 cm. In certain embodiments, the bendable tube section 12 has a length ranging from about 1 to about 2 cm. In certain embodiments, the bendable tube section 12 has a length of about 1 or about 2 cm.

In certain embodiments, the bendable tube section 12 has an I.D. that is the same as that of the first straight tube section 11 or the second straight tube section 13.

In certain embodiments, the bendable tube section 12 has an I.D. ranging from about 8 to about 20 French, from about 10 to about 15 French, or from about 11 to about 14 French. In certain embodiments, the bendable tube section 12 has an I.D. ranging from about 8 to about 20 French. In certain embodiments, the bendable tube section 12 has an I.D. ranging from about 10 to about 15 French. In certain embodiments, the bendable tube section 12 has an I.D. ranging from about 11 to about 14 French. In certain embodiments, the bendable tube section 12 has an I.D. of about 11, about 12, about 13, or about 14 French.

In certain embodiments, the bendable tube section 12 has an O.D. that is no more than about 2 French larger than that of the first straight tube section 11 or the second straight tube section 13. In certain embodiments, the bendable tube section 12 has an O.D. that is no more than about 1 French larger than that of the first straight tube section 11 or the second straight tube section 13. In certain embodiments, the bendable tube section 12 has an O.D. that is the same as that of the first straight tube section 11 or the second straight tube section 13.

In certain embodiments, the bendable tube section 12 has an outer diameter (O.D.) ranging from about 9 to about 23 French, from about 11 to about 18 French, or from about 12 to about 17 French. In certain embodiments, the bendable tube section 12 has an O.D. ranging from about 9 to about 23 French. In certain embodiments, the bendable tube section 12 has an O.D. ranging from about 11 to about 18 French. In certain embodiments, the bendable tube section 12 has an O.D. ranging from about 12 to about 17 French. In certain embodiments, the bendable tube section 12 has an O.D. of about 12, about 13, about 14, about 15, about 16, or about 17 French.

In certain embodiments, the second straight tube section 13 has a length that is no greater than that of the first straight tube section 11. In certain embodiments, the second straight tube section 13 has a length that is less than that of the second straight tube section 13.

In certain embodiments, the second straight tube section 13 has a length ranging from about 1 to about 5 cm, from about 2 to about 4 cm, or from about 2 to about 3 cm. In certain embodiments, the second straight tube section 13 has a length ranging from about 1 to about 5 cm. In certain embodiments, the second straight tube section 13 has a length ranging from about 2 to about 4 cm. In certain embodiments, the second straight tube section 13 has a length ranging from about 2 to about 3 cm. In certain embodiments, the second straight tube section 13 has a length of about 2 or about 3 cm.

In certain embodiments, the second straight tube section 13 has an I.D. that is the same as that of the first straight tube section 11 or the bendable tube section 12.

In certain embodiments, the second straight tube section 13 has an I.D. ranging from about 8 to about 20 French, from about 10 to about 15 French, or from about 11 to about 14 French. In certain embodiments, the second straight tube section 13 has an I.D. ranging from about 8 to about 20 French. In certain embodiments, the second straight tube section 13 has an I.D. ranging from about 10 to about 15 French. In certain embodiments, the second straight tube section 13 has an I.D. ranging from about 11 to about 14 French. In certain embodiments, the second straight tube section 13 has an I.D. of about 11, about 12, about 13, or about 14 French.

In certain embodiments, the second straight tube section 13 has an O.D. that is no greater than that of the first straight tube section 11. In certain embodiments, the second straight tube section 13 has an O.D. that is the same as that of the first straight tube section 11.

In certain embodiments, the second straight tube section 13 has an O.D. ranging from about 9 to about 22 French, from about 11 to about 17 French, or from about 12 to about 16 French. In certain embodiments, the second straight tube section 13 has an O.D. ranging from about 9 to about 22 French. In certain embodiments, the second straight tube section 13 has an O.D. ranging from about 11 to about 17 French. In certain embodiments, the second straight tube section 13 has an O.D. ranging from about 12 to about 16 French. In certain embodiments, the second straight tube section 13 has an O.D. of about 12, about 13, about 14, about 15, or about 16 French.

The inner tube 2 has a front end and a tail end. The distance between the front and tail ends defines the length of the inner tube 2. In certain embodiments, the inner tube 2 has a length that is greater than that of the sheath tube 1. In certain embodiments, the inner tube 2 has a length that is about 1, about 2, about 3, about 4, or about 5 cm greater than that of the sheath tube 1.

In certain embodiments, the inner tube 2 has a length ranging from about 20 to about 65 cm, from about 20 to about 60 cm, from about 30 to about 55 cm, or from about 30 to about 50 cm. In certain embodiments, the inner tube 2 has a length ranging from about 20 to about 65 cm. In certain embodiments, the inner tube 2 has a length ranging from about 20 to about 60 cm. In certain embodiments, the inner tube 2 has a length ranging from about 30 to about 55 cm. In certain embodiments, the inner tube 2 has a length ranging from about 30 to about 50 cm. In certain embodiments, the inner tube 2 has a length of about 37, about 40, about 42, about 45, about 47, or about 50 cm.

In certain embodiments, the inner tube 2 has an I.D. that is sufficient to allow a guidewire passing through.

In certain embodiments, the inner tube 2 has an O.D. that is no greater than that of the first straight tube section 11, the bendable tube section 12, or the second straight tube section 13. In certain embodiments, the inner tube 2 has an O.D. ranging from about 8 to about 20 French, from about 10 to about 15 French, or from about 11 to about 14 French. In certain embodiments, the inner tube 2 has an O.D. ranging from about 8 to about 20 French. In certain embodiments, the inner tube 2 has an O.D. ranging from about 10 to about 15 French. In certain embodiments, the inner tube 2 has an O.D. ranging from about 11 to about 14 French. In certain embodiments, the inner tube 2 has an O.D. of about 11, about 12, about 13, or about 14 French.

In certain embodiments, the inner tube 2 is made from a plastic material. In certain embodiments, the inner tube 2 is made from a fluorinated ethylene propylene (FEP) copolymer, latex, a nylon, a polyether block amide, a polyethylene, a polyethylene terephthalate (PET), a polytetrafluoroethylene (PTFE), a polyurethane, or a silicone.

In one embodiment, provided herein is a method, not forming part of the present invention, of providing a working channel for an operation, comprising the steps of:
a. inserting a bendable ureteral access sheath provided herein into a urethra to an operating position; and
b. retracting the inner tube 2 from the urethra such that the sheath tube 1 remains in the urethra as a working channel.

In another embodiment, provided herein is a method, not forming part of the present invention, of providing a working channel for an operation, comprising the steps of:
a. inserting a bendable ureteral access sheath provided herein into a urethra along a guidewire to an operating position; and
b. retracting the inner tube 2 from the urethra such that the sheath tube 1 remains in the urethra as a working channel.

The examples set forth above are provided to give those of ordinary skill in the art with a complete disclosure and description of how to make and use the claimed embodiments and are not intended to limit the scope of what is disclosed herein. Modifications that are obvious to persons of skill in the art are intended to be within the scope of the following claims.

## Claims

1. A bendable ureteral access sheath comprising a sheath tube (1), an inner tube (2), and a sheath tube hub (3); hub; wherein the sheath tube is sleeved on the outside of the inner tube; wherein the sheath tube hub is connected to the tail end of the sheath tube; and wherein the sheath tube comprises a first straight tube section (11), a bendable tube section (12), and a second straight tube section (13), where the first straight tube section, bendable tube section, and second straight tube section are connected to each other sequentially, where the first and second straight tube sections each comprise reinforcing steel wires (14) in the wall of each straight tube section, where the first and second straight tube sections each are kink-resistant, and the bendable tube section is of a flexible structure and comprises annular reinforcing rings (15) in a foldable tube structure, **characterized in that**
said annular reinforcing rings are made of steel and **in that**
said foldable tube structure is corrugated.

2. The bendable ureteral access sheath of claim 1, wherein the reinforcing steel wires are configured longitudinally in the tube walls of the first and second straight tube sections at equal intervals.

3. The bendable ureteral access sheath of claim 1 or 2, wherein the sheath tube is made of a plastic material *via* injection molding, and the sheath tube has interior and outer walls each coated with a hydrophilic coating.

4. The bendable ureteral access sheath of any one of claims 1 to 3, wherein the inner tube is made of a polytetrafluoroethylene having a certain bendability.

5. The bendable ureteral access sheath of any one of claims 1 to 4, wherein the bendable tube section has a length ranging from 1 to 2 cm and the first straight tube section has a length ranging from 2 to 3 cm.

## Patentansprüche

1. Biegsame Ureterzugangshülle, die ein Hüllrohr (1), ein Innenrohr (2) und eine Hüllrohrnabe (3) umfasst;
wobei das Hüllrohr an der Außenseite des Innenrohrs ummantelt ist; wobei die Hüllrohrnabe mit dem hinteren Ende des Hüllrohrs verbunden ist; und wobei das Hüllrohr einen ersten geraden Rohrabschnitt (11), einen biegsamen Rohrabschnitt (12) und einen zweiten geraden Rohrabschnitt (13) umfasst, wobei der erste gerade Rohrabschnitt, der biegsame Rohrabschnitt und der zweite gerade Rohrabschnitt nacheinander miteinander verbunden sind, wobei der erste und der zweite gerade Rohrabschnitt jeweils Verstärkungsstahldrähte (14) in der Wand jedes geraden Rohrabschnitts umfassen, wobei der erste und der zweite gerade Rohrabschnitt jeweils knickfest sind, und der biegbare Rohrabschnitt von flexibler Struktur ist und ringförmige Verstärkungsringe (15) in einer faltbaren Rohrstruktur umfasst, **dadurch gekennzeichnet, dass**
die ringförmigen Verstärkungsringe aus Stahl hergestellt sind, und dadurch, dass
die faltbare Rohrstruktur gewellt ist.

2. Biegsame Ureterzugangshülle nach Anspruch 1, wobei die Verstärkungsstahldrähte in gleichen Abständen längs in den Rohrwänden des ersten und des zweiten geraden Rohrabschnitts konfiguriert sind.

3. Biegsame Ureterzugangshülle nach Anspruch 1 oder 2, wobei das Hüllrohr durch Spritzguss aus einem Kunststoffmaterial hergestellt wird und das Hüllrohr Innen- und Außenwände aufweist, die jeweils mit einer hydrophilen Beschichtung beschichtet sind.

4. Biegsame Ureterzugangshülle nach einem der Ansprüche 1 bis 3, wobei das Innenrohr aus einem Polytetrafluorethylen hergestellt wird, das eine gewisse Biegsamkeit aufweist.

5. Biegsame Ureterzugangshülle nach einem der Ansprüche 1 bis 4, wobei der biegsame Rohrabschnitt eine Länge im Bereich von 1 bis 2 cm aufweist und der erste gerade Rohrabschnitt eine Länge im Bereich von 2 bis 3 cm aufweist.

## Revendications

1. Gaine d'accès urétéral pliable comprenant un tube de gaine (1), un tube interne (2) et un moyeu de tube de gaine (3) ;
dans laquelle le tube de gaine est manchonné sur l'extérieur du tube interne ; dans laquelle le moyeu de tube de gaine est relié à l'extrémité arrière du tube de gaine ; et dans laquelle le tube de gaine comprend une première section de tube droite (11), une section de tube pliable (12) et une seconde section de tube droite (13), la première section de tube droite, la section de tube pliable et la seconde section de tube droite étant reliées les unes aux autres séquentiellement, les première et seconde sections de tube droites comprenant chacune des fils d'acier de renforcement (14) dans la paroi de chaque section de tube droite, les première et seconde sections de tube droites étant chacune résistantes au vrillage, et la section de tube pliable est d'une structure flexible et comprend des anneaux de renforcement annulaires (15) dans une structure de tube pliable, **caractérisée en ce que**
lesdits anneaux de renforcement annulaires sont en acier, et **en ce que**
ladite structure de tube pliable est ondulée.

2. Gaine d'accès urétéral pliable selon la revendication 1, dans laquelle les fils d'acier de renforcement sont configurés longitudinalement dans les parois de tube des première et seconde sections de tube droites à des intervalles égaux.

3. Gaine d'accès urétéral pliable selon la revendication 1 ou 2, dans laquelle le tube de gaine est réalisé en une matière plastique par moulage par injection, et le tube de gaine présente des parois intérieure et extérieure recouvertes chacune d'un revêtement hydrophile.

4. Gaine d'accès urétéral pliable selon l'une quelconque des revendications 1 à 3, dans laquelle le tube interne est réalisé en un polytétrafluoroéthylène présentant une certaine aptitude à la flexion.

5. Gaine d'accès urétéral pliable selon l'une quelconque des revendications 1 à 4, dans laquelle la section de tube pliable présente une longueur allant de 1 à 2 cm et la première section de tube droite présente une longueur allant de 2 à 3 cm.
